# EUROPEAN PATENT APPLICATION

(11) **EP 4 785 861 A2**
(43) Date of publication of application: **05.08.2026**
(21) Application number: 26180004.9
(22) Date of filing: 11.03.2022
(51) Int. Cl.: A61B 5/0538

(54) **METHOD AND SYSTEM FOR MEASURING A DEGREE OF MUSCLE DISPLACEMENT AND USE OF THE METHOD AND SYSTEM IN TREATING OBSTRUCTIVE SLEEP APNEA**

(62) Divisional of application: 22161739.2
(71) Applicant: Nyxoah SA, 1435 Mont-St-Guibert (BE)
(72) Inventor: Kohn, Sarah, 7174661 Modiin (IL); Tsukran, Roi, 7575721 Rishon Le-Zion (IL)
(74) Representative: Brantsandpatents bv

(57) **Abstract**

The invention described herein refers to a method for measuring a degree of airway opening, e.g. of muscle displacement, to a system for implementing said method as well as to a use of the method and system in obstructive sleep apnea therapy.

## Description

The invention described herein refers to a method for measuring a degree of muscle displacement, to a system for implementing said method and to a use of the method and system in obstructive sleep apnea therapy.

Neural modulation, i.e. electrical stimulation of nerves, is well-known in the prior art as a reliable and effective type of medical treatment. It presents the opportunity to tackle many physiological conditions and disorders by interacting with the body's own natural neural processes. Neural modulation includes inhibition (e.g. blockage), stimulation, modification, regulation, or therapeutic alteration of activity, electrical or chemical, in the central, peripheral, or autonomic nervous system. By modulating the activity of the nervous system, several different goals may be achieved. For instance, motor neurons may be stimulated at appropriate times to cause muscle contractions. Further, sensory neurons can be blocked to relieve pain or stimulated to provide a signal to a subject. In yet other examples, modulation of the autonomy nervous system may be used to adjust various involuntary physiological parameters, such as heart rate and blood pressure. Neural modulation may provide the opportunity to treat several diseases or physiological conditions. Various devices and techniques have been used in attempts to provide optimum stimulation of a tissue of interest.

Within the content of this disclosure, the expressions "nerve stimulation", "neural stimulation", "nerve modulation" and "neural modulation" are used synonymously unless something else is apparent from the respective context. In general, the above expressions refer to the process of generating an electric field in the vicinity of a nerve or a group of nerves in order to cause contraction of one or more muscles associated to said nerves. Likewise, the expressions "subject" and "patient" are used synonymously unless something else is apparent from the respective context. Both terms refer to a person potentially suffering from OSA.

One of the conditions to which neural modulation can be applied to is obstructive sleep apnea (OSA), a respiratory disorder characterized by recurrent episodes of partial or complete obstruction of the upper airway during sleep. One of the major causes of OSA is the inability of the tongue muscles to resist negative inspiratory pressure in the pharynx due to the sleep-related loss in muscle tone. As the tongue is pulled backwards, it obstructs the upper airway, decreasing ventilation and lowering lung and blood oxygen levels. Stimulation of the hypoglossal nerve ("Hypoglossal Nerve Stimulation" or HGNS) causes the tongue muscles to contract, thereby maintaining an open, unobstructed airway. When a person without OSA is sleeping, the pharyngeal muscles - a group of muscles that form the pharynx - relax and gradually collapse, narrowing the airway. Narrowing of the airway in turn limits the effectiveness of the sleeper's breathing, causing a rise in CO2 levels in the blood of the sleeper. The increase in CO2 results in the pharyngeal muscles contracting to open the airway to restore proper breathing. The larger of the pharyngeal muscles responsible for upper airway dilation is the genioglossus muscle, which is one of several different muscles in the tongue.

The genioglossus muscle is responsible for the forward movement of the tongue as well as for the stiffening of the anterior pharyngeal wall. In patients with OSA, the neuromuscular activity of the genioglossus muscle is decreased compared to normal individuals, accounting for insufficient response and contraction to open the airway as compared to a normal individual. This lack of response contributes to a partial or total airway obstruction, which significantly limits the effectiveness of the sleeper's breathing. In OSA patients, there are often several airway obstruction events during the night. Because of the obstruction, there is a gradual decrease of O2 levels in the blood (hypoxemia). Hypoxemia leads to night time arousals, which may be registered by EEG, showing that the brain awakes from any stage of sleep to a short arousal. During the arousal, there is a conscious breath or gasp, which resolves the airway obstruction. An increase in sympathetic tone activity rate through the release of hormones such as epinephrine and noradrenaline also often occurs as a response to hypoxemia. Because of the increase in sympathetic tone, the heart enlarges in an attempt to pump more blood and increase the blood pressure and heart rate, further arousing the patient after the resolution of the apnea event, as the patient returns to sleep, the airway collapses again, leading to further arousals.

In order to quantify the efficacy of neural modulation as a therapeutic method (in particular HGNS) as well as for analytical purposes, it is important that therapy response is closely monitored. This is usually achieved via through monitoring of nasal and/or oral airflow of the subject, e.g. with the use of PSG (sleep study or Polysomnography) or CPAP (Continuous Positive Airway Pressure) masks. Alternatively, patients can self-assess therapy efficacy during wakeful titration. Other methods may include visually observing airway opening or tongue protrusion to assess if the therapy resulted in the desired motor response. Further assessments of therapy response include drug-induced or wakeful endoscopy.

The above methods have several disadvantages. Solutions involving airflow are not suitable for seamless implementation in existing systems for neuro stimulation. Furthermore, using masks and the like may be perceived as annoying by the subject. As for self-assessment or visualization of airway opening or tongue protrusion, these methods can be highly subjective and are therefore subject to variating results or mistakes. Furthermore, even objective visual assessments merely provide qualitative results rather than quantitative.

The objective technical problem of the present disclosure is to eliminate the disadvantages of the prior art and to provide a method for measuring a degree of airway opening, e.g. of muscle displacement in order to facilitate quantification of the efficacy of neural modulation therapy. Furthermore, the method should improve electrical nerve stimulation in a patient associated with OSA.

### SUMMARY OF THE INVENTION

Main features of the method according to the disclosure are specified by claim 1. Special embodiments or beneficial variants of the method are depicted in claims 2 to 7. A system for implementing the method is specified by claims 8 to 9. Further, a use of the method is specified by claim 10.

According to a first aspect of the disclosure, the objective technical problem is solved by a method for measuring a degree of muscle collapse and/or contraction of a subject in response to electrical nerve stimulation, the method comprising:
- generating a modulation signal, the modulation signal comprising an electrical stimulation pattern;
- applying the modulation signal to at least one pair of electrodes associated with an implant unit implanted inside the subject's body;
- detecting a movement of one or more muscles related to the subject's airway in response to the modulation signal applied to the at least one pair of electrodes;
- assigning to the determined movement a value that is indicative of the degree of airway opening and/or obstruction, e.g. of muscle displacement.

With the method as described above, the efficacy of nerve stimulation during treatment of patients suffering from OSA, in particular the efficacy of stimulation of the hypoglossal nerve or of the ansa cervicalis, can be monitored in an easy and accurate manner. It is understood that the hypoglossal nerve innervates the genioglossus muscle and that the ansa cervicalis innervates the infrahyoid strap muscles, which are responsible for stiffening or collapsing the upper airway of a subject. The infrahyoid muscles include the sternohyoid muscle, the sternothyroid muscle, the omohyoid muscle, and the thyrohyoid muscle. Furthermore, if needed, the system allows for continuous monitoring over a desired time period, providing important information regarding the long-term effects of nerve stimulation therapy.

Muscle contraction in the sense of this disclosure may refer to muscle displacement, and these expressions are used synonymously unless indicated otherwise. Muscle contraction may in particular refer to (but is not limited to) displacement of the subject's tongue or of the other muscles associated to the subject's airway.

Movement of one or more muscles related to the subject's airway may, in the sense of this disclosure, also comprise movement of the chin of the subject, since contraction of one or more of the muscles involved in tongue movement will also result in the chin being moved. Thus, the degree of airway opening and/or of airway obstruction, e.g. of muscle displacement may also be determined by measurement of the subject's chin movement.

The modulation signal comprising the electrical stimulation pattern is preferably generated by an external device, which may be configured for placement underneath the subject's chin. In particular, the modulation signal may be generated by a control unit and/or by a processor, which can both be part of the external unit. The modulation signal may further be transmitted to an implant unit using a transmitting element, which may comprise an antenna or a coil associated with the control unit.

According to another embodiment, the control unit may be part of the implant unit implanted beneath the subject's skin, either instead of or in addition to a control unit of the external device. This way, the control unit may be located directly on the muscle to be monitored, e.g. on the genioglossus muscle or on one or more of the infrahyoid muscles, in particular on the sternohyiod or the sternothyroid muscle. In other words, location of the control unit it is not essential for the underlying method of this disclosure. Either way, the control unit may preferably be in electrical commutation with the at least one motion sensing unit.

The step of applying the modulation signal to at least one pair of electrodes may preferably be implemented by the implant unit. The implant unit may be implantable in a vicinity or proximity of any muscle associated with the subject's airway, e.g. the genioglossus muscle, which is innervated by the hypoglossal nerve. The implant unit may comprise an electric circuit which receives the electrical stimulation pattern through an electrical communication between the external device and the implant unit and in turn generates an electric field via at the least one pair of electrodes associated with the implant unit.

The step of determining a movement of one or more muscles related to the subject's airway opening in response to the modulation signal may preferably be implemented by a motion sensing unit. Likewise, the muscles related to the subject's airway opening are also responsible for airway obstruction, which can be detected using the method described herein. The method may further be characterized in that the at least one motion sensing unit comprises at least one inertial measurement unit (IMU) and/or at least one strain gauge. According to an exemplary embodiment, movement of one or more muscles related to the subject's airway opening and/or obstruction may also be determined by detecting movement of the chin of the subject, since movement of the muscles related in the subject's airway opening and/or airway obstruction and in particular a movement of a subject's tongue will also result in movement of a subject's chin.

Using the presented method, the actual physiological effect to be achieved via stimulation therapy, i.e. a contraction (or non-contraction) of the targeted muscle or group of muscles, can be monitored rather than the symptoms of the illness or disorder. As the latter may not immediately be improved by the therapy in some cases, they may be misleading when monitored and used as basis for adjusting the stimulation. For example, if a patient or subject suffers from a disorder like central sleep apnea (CSA), a measurement of the airflow in response to nerve stimulation therapy will indicate an apnea event. This would suggest non-effectiveness of the therapy and potentially lead to the decision to increase stimulation intensity. However, since stimulation therapy will not be able to prevent an apnea event in CSA in the first place, increasing or decreasing the stimulation intensity (or adjusting any other of the stimulation parameters) will not affect the condition of the patient with regards to the sleep apnea disorder. Therefore, airflow is no viable indicator for determining efficacy of nerve stimulation.

With the method described herein, the physiological condition that is directly affected by the underlying nerve stimulation therapy, i.e. muscle contraction, is monitored. This way, it is possible to obtain accurate information regarding therapy efficacy. Based on the determined degree of muscle displacement in response to stimulation with a defined set of stimulation parameters, it is further possible to adjust those parameters in order to improve therapy efficacy. For example, if a set group of stimulation parameters is not sufficient to cause tongue protrusion and open the airway, the underlying stimulation parameters may be adjusted accordingly, e.g. by increasing stimulation intensity.

According to a further embodiment of the method, a threshold degree of airway opening and/or airway obstruction (e.g. of muscle displacement) may be pre-defined. In such a case, a value indicative of airway opening will only be assigned to the determined muscle movement if the threshold degree of airway opening is surpassed. Airway opening may for example comprise tongue protrusion caused by innervation of the genioglossus muscle, or widening of the upper airway caused by innervation of one or more of the infrahyiod muslces.

According to one embodiment disclosed herein, the method further comprises the step: adjusting at least one parameter of the electrical stimulation pattern in response to the value indicative of the degree of airway opening, e.g. of muscle displacement. This way, feedback-based or closed loop stimulation method is provided, wherein the efficacy of an ongoing stimulation session is determined by measuring the degree of displacement of the subject's muscle in response to the associated nerve stimulation and then adjusting the stimulation parameters in response to the measured degree of airway opening and/or airway obstruction. The degree of airway opening and/or airway obstruction can for example be determined by detecting movement of the muscles associated with the tongue (i.e. collapse or protrusion) or by detecting movement of the subject's chin. Adjustment of the at least one stimulation parameter can occur automatically or by hand (e.g. through a physician or through the subject themselves) or both. In case of automatic adjustment, the at least one stimulation parameter can be changed after a set time delay or more or less instantly (i.e. in real-time). This process may be automatically repeated in an iterative manner until one of the automatic adjustments of the stimulation parameters results in determination of a value that is indicative of muscular contraction associated with a desired degree of airway opening and/or airway obstruction.

In order to achieve a feedback-based or closed loop stimulation session, the control unit may be configured to perform logical operations such as comparing the detected degree of movement (or non-movement) of one of the muscles related to the subject's airway to a pre-defined value and adjust one or more of the stimulation parameters accordingly. For example, in case the detected degree of tongue displacement or chin movement is below the pre-determined value, the control unit may be configured to increase stimulation intensity. Another example is, in case the degree of tongue collapse is above a pre-determined value, i.e. resulting in an obstruction of the subject's airway, the control unit may be configured to induce stimulation.

Advantageously, the movement of the muscles related to the subject's airway may comprise a muscle contraction, a change of the tongue's position and/or a change of the tongue's spatial orientation within the subject, and/or movement of the subject's chin. In particular but not exclusively, muscle contraction may refer to contraction of the genioglossus muscle, especially when innervated by the hypoglossal nerve.

It is also possible that the movement of the one or more muscles related to the subject's tongue is determined using at least one motion sensing unit. The method may further be characterized in that the at least one motion sensing unit comprises at least one inertial measurement unit and/or at least one strain gauge. According to another embodiment, the inertial measurement unit may comprise at least one accelerometer and/or at least one gyroscope and/or at least one magnetometer. Preferably, at least one accelerometer may be implemented, wherein the accelerometer may comprise a single axis accelerometer or a multiple axis accelerometer.

The at least one parameter may comprise a pulse train amplitude, a pulse train length, a single pulse frequency, a single pulse duration, a hold duration, a pulse train interval, a duty cycle, a delay time, a ramp duration, a step-down amplitude, a ramp at train onset duration, and/or a confirmatory pulse/train amplitude. The stimulation parameters may be adjusted automatically based on the value assigned to the measured degree of airway opening and/or airway obstruction, or the parameters can be adjusted by a physician or by the patients themselves upon receiving information that the current stimulation parameters are not sufficient.

According to another aspect of this invention, a system for measuring a degree of airway opening and/or airway obstruction, e.g. of muscle displacement of a subject is presented, the system comprising:
- an implant unit configured for implantation in the vicinity or proximity of any muscle associated with the subject's airway, e.g. the genioglossus muscle or one of the infrahyoid strap muscles, the implant unit comprising at least one pair of electrodes;
- an external device configured for communication with the implant unit;
- at least one motion sensing unit for determining movement of one or more muscles related to the subject's airway in response to a modulation signal applied to the at least one pair of electrodes.

The system may preferably be configured for implementing the method described herein.

In addition to the above, the system may comprise a control unit. The control unit may be part of the external device. In that case, the control unit may transmit modulation or stimulation input signals, e.g. the stimulation parameters, and power to the implant unit. The control unit may further receive the determined data corresponding to the movement of one or more muscles related to the subject's tongue determined by the motion sensing unit, and assign a value to the degree of airway opening and/or airway obstruction. According to a preferred embodiment, the control unit is also able to adjust at least one of the stimulation parameters in response to the value indicative of the degree of airway opening and/or airway obstruction, e.g. of muscle displacement.

Accordingly, the control unit may comprise one or more processors, which may include any electric circuit that configured to perform a logic operation on at least one input variable. The at least one processor of the control unit may therefore include one or more integrated circuits, microchips, microcontrollers, and microprocessors, which may be all or part of a central processing unit (CPU), a digital signal processor (DSP), a field programmable gate array (FPGA), or any other circuit known to those skilled in the art that may be suitable for executing instructions or performing logic operations.

The implant unit may be configured for implantation in a location that permits it to modulate a nerve (e.g. the hypoglossal nerve). The implant unit may be located in a subject such that intervening tissue exists between the implant unit and the nerve to be modulated. Intervening tissue may include muscle tissue, connective tissue, organ tissue, or any other type of biological tissue. Thus, location of the implant unit does not require immediate contact with the nerve for effective neuromodulation. The implant unit may also be located directly adjacent to nerve, such that no intervening tissue exists.

In treating OSA, the implant unit may be located on the genioglossus muscle of the patient or subject. Such a location is suitable for modulation of the hypoglossal nerve, branches of which run inside and innervate the genioglossus muscle. However, the implant unit may also be configured for placement in other locations.

The external device may be configured for location external to a patient, either directly contacting the skin or close to the skin of the patient. The external device may further be configured to be affixed to the patient, for example, by adhering to the skin of the patient, or through a band or other device configured to hold the external device in place. Adherence to the skin of the external unit may occur such that it is in the vicinity or in the proximity of the location of the implant unit.

The external device may be configured for fixation to the patient. For example, the external device may be configured for placement underneath the subject's chin and/or on the front of patient's neck. The suitability of placement locations may be determined by communication between external device and implant unit. The external device may comprise a housing, wherein the housing may be any suitable container configured for retaining electrical components. In addition, the housing may be any suitable size and/or shape and may be rigid or flexible. Examples of housings for the external device may include one or more of patches, buttons, or other receptacles having varying shapes and dimensions and constructed of any suitable material. The external device may be configured to adhere to a desired location. Accordingly, in some embodiments, at least one side of the housing may include an adhesive material. The adhesive material may include a biocompatible material and may allow for a patient to adhere the external unit to the desired location and remove the external device upon completion of use. The adhesive may be configured for single or multiple uses of the external unit. Suitable adhesive materials may include, but are not limited to biocompatible glues, starches, elastomers, thermoplastics, and emulsions.

According to one embodiment, the at least one motion sensing unit may be part of the implant unit and/or the at least one motion sensing unit may be part of the external device. It is also possible that both the implant unit and the external device comprise a motion sensing unit. If this is the case, the motion sensing units can be different form each other, i.e. the motion sensing unit of the internal unit may be an strain gauge and the motion sensing unit of the external device may be an accelerometer. For example, if the motion sensing unit is part of the external device, the motion sensing unit may preferably be a IMU configured to detect chin movement, since the external device is preferably located underneath the subject's chin and since a stimulation event resulting in contraction of one or more muscles related to tongue movement will also result in movement of the subject's chin.

If the motion sensing unit is part of the implant unit, the motion sensing unit may preferably comprise a strain gauge, either as a stand-alone component or together with an IMU, as a strain gauge is suitable for detecting and measuring muscle contraction.

According to another aspect of the disclosure, a use of the method disclosed herein for treatment of obstructive sleep apnea is presented. The treatment of obstructive sleep apnea, for which the method can be used, may for example comprise the following steps:
- receiving a modulation signal at an implant unit implanted at an internal location on an underside of a subject's chin (e.g.in the vicinity of the genioglossus muscle);
- applying the modulation signal to at least one pair of electrodes associated with the implant unit to generate an electric field;
- and causing modulation of a hypoglossal nerve or of an ansa cervicalis of the subject in response to the electric field generated by the at least one pair of electrodes, wherein the modulation of the hypoglossal nerve may be confined to a medial branch of the hypoglossal nerve and may be initiated from a single modulation site along the medial branch.

Therefore, the at least one pair of modulation electrodes may be configured for implantation through derma on the underside of the subject's chin and for location proximate to terminal fibers of the medial branch of the subject's hypoglossal nerve. In addition, the implantable unit and the electrodes may be configured to cooperate in order to generate an electric field adapted to modulate one or more of the terminal fibers of the medial branch of the hypoglossal nerve.

Any one of the embodiments, examples or features disclosed herein may be used in combination or separately and in conjunction with any one of the aspect of the disclosed subject matter.

### BRIEF DESCRIPTION OF THE DRAWINGS

The accompanying drawings, which are incorporated in and constitute a part of this specification, illustrate several examples of the disclosed subject matter. The drawings show the following:
Fig. 1 shows a schematic depiction of the system disclosed herein according to a first embodiment;
Fig. 2 shows a schematic depiction of a system according to a second embodiment;
Fig. 3 shows a schematic depiction of a system according to a third embodiment.

### DETAILED DESCRIPTION OF THE DRAWINGS

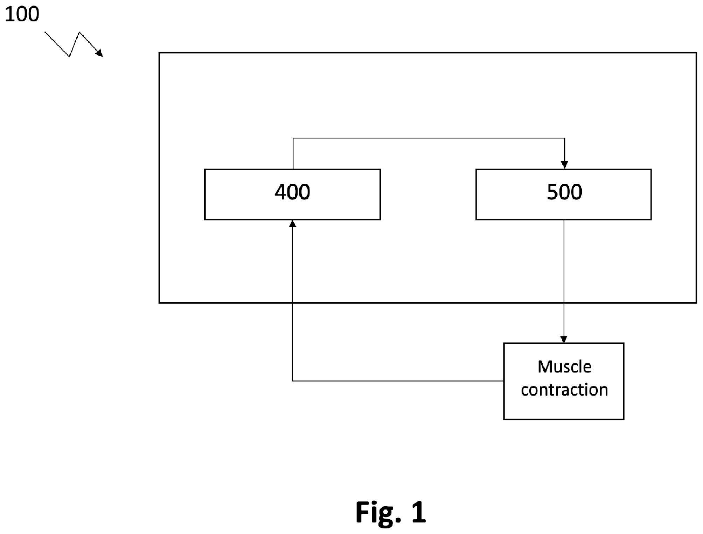

Fig. 1 shows a schematic depiction of a system 100 for measuring a degree of airway opening and/or airway obstruction, e.g. of muscle displacement of a subject in response to electrical nerve stimulation, wherein said muscle is part of the group of muscles responsible for the subject's breathing, e.g. the tongue, the genioglossus muscle or one or more of the infrahyoid strap muscles. The flow diagram of Fig. 1 may serve as representation of the underlying concept of the method. According to Fig. 1, a neuro stimulation system 100 configured for implementing the method comprises a control unit 500 and a motion sensing unit 400. Furthermore, the system 100 may preferably comprise an implant unit 200 (not shown) configured for implantation in the proximity of any muscle associated with the subject's airway, e.g. the genioglossus muscle, and an external device 300 (not shown) configured for electrical communication with the implant unit 200.

The implant unit 200 of the system 100 with which the method may be carried out, may be implanted in a vicinity to the genioglossus muscle, which is innervated by the hypoglossal nerve, or to one of the infrahyoid muscles, which are innervated by the ansa cervicalis. The implant unit 200 may further comprise a transmitting element, which may comprise a secondary antenna or a coil, and an electric circuit which receives an electrical stimulation pattern through the electrical communication between the external device 300 and the implant unit 200. Upon receiving the electrical stimulation pattern, the electric circuit may generate an electric field via at the at least one pair of electrodes associated with the implant unit 200. If the electric field is strong enough and/or close enough to the nerve, the nerve will be stimulated and innervate the associated muscle, which will in turn contract. The specifications of the implant unit 200 are not limited to a certain embodiment as long as the implant unit 200 is configured for use with the method described herein.

The external device 300 of the system 100 with which the method may be carried out, may be configured for location external to a patient, in particular underneath the subject's chin and/or on the front of patient's neck. The external device 300 may therefore be configured for affixation to the patient, for example, by adhering to the skin of the patient, or through a band or other device configured to hold the external device in place. The external device 300 may preferably comprise a housing, wherein the housing can be any suitable container configured for retaining electrical components. In addition, the housing may be any suitable size and/or shape and may be rigid or flexible. Examples of housings for the external device 300 may include one or more of patches, buttons, or other receptacles having varying shapes and dimensions and constructed of any suitable material. The specifications of the external device 300 are not limited to a certain embodiment as long as the implant unit 200 is configured for use with the method described herein.

In order to determine a movement (i.e. a contraction) of one or more muscles related to the subject's airway in response to the electric field applied by the implant unit 200, the system 100 comprises a motion sensing unit 400, which comprises at least one inertial measurement unit 401 and/or at least one strain gauge. The inertial measurement unit may comprise at least one accelerometer and/or at least one gyroscope and/or at least one magnetometer.

Lastly, the system 100 according to Fig. 1 may comprise a control unit 500, for example as part of the external device 300. The control unit 500 may transmit modulation or stimulation input signals, e.g. the stimulation parameters, and power to the implant unit 200.

According to the method depicted in Fig. 1, the control unit 500 may also receive data corresponding to the movement of the one or more muscles related to the subject's breathing or airway determined by the motion sensing unit 400, and assign to it a value relating to a degree of airway opening and/or airway obstruction. According to a preferred embodiment of the method, the control unit 500 is also able to adjust at least one of the stimulation parameters in response to the value indicative of the degree of airway opening and/or airway obstruction. Accordingly, the control unit 500 may comprise one or more processors, which may include any electric circuit that configured to perform a logic operation on at least one input variable. The at least one processor of the control unit 500 may for example include one or more integrated circuits, microchips, microcontrollers, and microprocessors, which may be all or part of a central processing unit (CPU), a digital signal processor (DSP), a field programmable gate array (FPGA), or any other circuit known to those skilled in the art that may be suitable for executing instructions or performing logic operations. This way, a method for feedback-based or closed loop neuro stimulation is provided, wherein the efficacy of an ongoing stimulation session is determined by measuring the degree of airway opening and then automatically adjusting the stimulation parameters in response to the measured degree of airway opening. The degree of protrusion of the subject's tongue can for example be determined by detecting movement of the muscle associated with the tongue or by detecting movement of the subject's chin. Automatic adjustment may comprise the control unit 500 receives a value indicative of tongue movement from the motion sensing unit 400 and thereupon adjusts at least one stimulation parameter, either after a set time delay or more or less instantly (i.e. in real-time).

In accordance with Fig. 1, the control unit 500 can be part of the external device 300, of the implant unit 200, or of both the external device 300 and the implant 200.

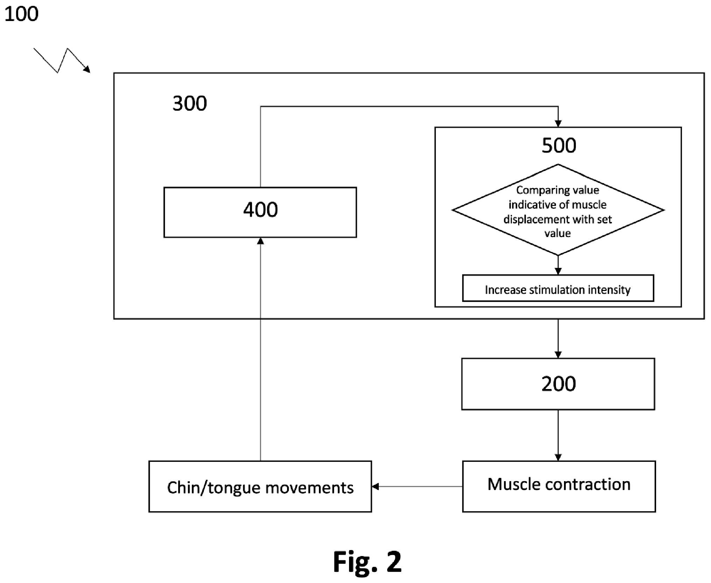

Fig. 2 shows a schematic depiction of a system 100 according to a second embodiment for measuring a degree of airway opening and/or airway obstruction, e.g. muscle displacement of a subject in response to electrical nerve stimulation. In comparison to the system of Fig. 1, the system 100 of Fig. 2 is further specified in that both the motion sensing unit 400 and the control unit 500 are shown as parts of the external device 300. In this case, the motion sensing unit 400 may preferably comprise at least one inertial measuring unit, e.g. an accelerometer.

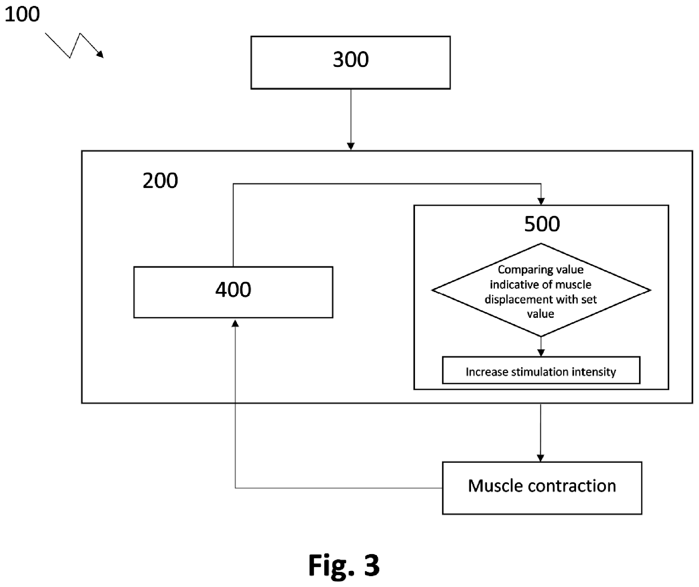

Fig. 3 depicts a flow diagram of a third embodiment of a system 100 for measuring a degree of airway opening, e.g. displacement of a subject in response to electrical nerve stimulation. The system 100 of Fig. 3 is further specified in that both the motion sensing unit 400 and the control unit 500 are shown as parts of the implant unit 200. In this case, the motion sensing unit 400 may preferably comprise at least one strain unit configured for detecting muscle contraction.

The invention is not limited to one of the embodiments described herein but may be modified in numerous other ways.

All features disclosed by the claims, the specification and the figures, as well as all advantages, including constructive particulars, spatial arrangements and methodological steps, can be essential to the invention either on their own or by various combinations with each other.

### LIST OF REFERENCE NUMERALS

- 100: System
- 200: Implant unit
- 300: External Device
- 400: Motion sensing unit
- 500: Control unit

## Claims

1. A method for measuring a degree of muscle contraction of a subject in response to electrical nerve stimulation, the method comprising:
- generating a modulation signal, the modulation signal comprising an electrical stimulation pattern;
- applying the modulation signal to at least one pair of electrodes associated with an implant unit implanted inside the subject's body;
- detecting movement of one or more muscles related to the subject's airway in response to the modulation signal applied to the at least one pair of electrodes;
- assigning to the determined movement a value that is indicative of the degree of airway opening and/or of airway obstruction.

2. The method according to 1 wherein the method further comprises:
- adjusting at least one parameter of the electrical stimulation pattern in response to the value indicative of the degree of airway opening and/or airway obstruction.

3. The method according to one of claims 1 or 2, **characterized in that** the movement of the muscles related to the subject's airway comprises an airway muscle contraction, a change of position of the subject's tongue and/or a change of the tongue's spatial orientation within the subject.

4. The method according to any of the preceding claims, **characterized in that** the movement of the one or more muscles related to the subject's airway is determined using at least one motion sensing unit.

5. The method according to claim 4, **characterized in that** the at least one motion sensing unit comprises at least one inertial measurement unit and/or at least one strain gauge.

6. The method according to claim 5, **characterized in that** the inertial measurement unit comprises at least one accelerometer and/or at least one gyroscope and/or at least one magnetometer.

7. The method according to any one of claims 2 to 6, **characterized in that** at least one parameter comprise a pulse train amplitude, a pulse train length, a single pulse frequency, a single pulse duration, a hold duration, a pulse train interval, a duty cycle, a delay time, a ramp duration, a step-down amplitude, a ramp at train onset duration, and/or a confirmatory pulse/train amplitude.

8. A system for implementing the method according to any of the preceding claims, the system comprising:
- an implant unit configured for implantation in the vicinity of the any muscle associated with the subject's airway opening and/or airway obstruction;
- an external device configured for communication with the implant unit;
- at least one motion sensing unit.

9. The system of claim 8 wherein the at least one motion sensing unit is part of the implant unit and/or wherein the at least one motion sensing unit is part of the external device.

10. Use of the method according to any one of claims 1 to 7 in the treatment of obstructive sleep apnea.
